# FASCICULE DE BREVET EUROPEEN

(11) **EP 2 596 832 B1**
(45) Date de publication et mention de la délivrance du brevet: **27.11.2013**
(21) Numéro de dépôt: 12183718.1
(22) Date de dépôt: 10.09.2012
(51) Int. Cl.: A61N 1/37, A61N 1/39, A61B 5/046

(54) **Dispositif médical de type défibrillateur/cardioverteur implantable avec seuil de détection ventriculaire ajustable dynamiquement**
Medizinische Vorrichtung vom Typ implantierbarer Kardioverter/Defibrillator mit dynamisch anpassbarer ventrikulärer Erkennungsschwelle
Implantable cardioverter-defibrillator medical device with dynamically adjustable ventricular sensing threshold

(30) Priorité: 22.11.2011 FR 1160668
(43) Date de publication de la demande: 29.05.2013
(73) Titulaire: Sorin CRM SAS, 92140 Clamart (FR)
(72) Inventeur: Limousin, Marcel, 75014 Paris (FR); Vincent, Elodie, 92160 Antony (FR); Euzen, Marie-Anen, 91570 Bievres (FR)
(74) Mandataire: Dupuis-Latour, Dominique

(56) Documents cités:
- US-A1- 2002 165 587
- US-A1- 2004 015 192
- US-A1- 2009 326 600
- US-A1- 2011 196 247

## Description

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas du trouble du rythme détecté par le dispositif.

Elle concerne plus particulièrement ceux de ces dispositifs qui possèdent un mode de thérapie antitachycardique impliquant, pour mettre fin à une tachyarythmie, l'application contrôlée de chocs de défibrillation sous forme d'impulsions électriques de haute énergie (appareils dénommés "défibrillateurs/cardioverteurs implantables" ou "ICDs"), et/ou une stimulation antitachycardique de type ATP (*AntiTachycardia Pacing*) ou analogue.

La décision d'appliquer une thérapie est prise par un algorithme d'analyse du rythme cardiaque et de détection d'un épisode de tachyarythmie. Les différents troubles sont détectés et classifiés en fonction de plusieurs critères de discrimination tels que la fréquence ventriculaire, la stabilité des intervalles ventriculaires (intervalles RR), l'analyse de l'association auriculo-ventriculaire (révélée par la stabilité de l'intervalle PR), le mode de démarrage de la tachycardie (présence d'une accélération brusque et cavité d'origine, ventriculaire ou auriculaire), ou encore la morphologie du signal intracardiaque.

L'analyse du rythme, et donc la décision de délivrer ou non une thérapie, peut cependant être perturbée par des artefacts recueillis par la sonde.

En effet, ces implants sont sensibles à la détection de signaux d'origine non cardiaque dus à des problèmes liés à la sonde, à des interférences électromagnétiques, à la détection de myopotentiels, etc., phénomènes ci-après désignés sous le terme générique de "bruit".

Ces phénomènes sont susceptibles de générer des artefacts dont la détection par l'implant peut avoir des conséquences très graves, comme la création d'une pause ventriculaire (liée à l'inhibition de la stimulation ventriculaire), qui peut être symptomatique pour les patients dépendants, ou comme le déclenchement de la délivrance de chocs de défibrillation inappropriés.

Or l'application d'un choc de défibrillation chez un patient conscient est extrêmement douloureuse et angoissante, les énergies appliquées étant en effet très au-delà du seuil de la douleur. En outre, l'application d'un choc de défibrillation n'est pas dénuée d'effets secondaires sur le rythme cardiaque (risque d'apparition de troubles secondaires), sur l'intégrité fonctionnelle du myocarde, et de façon générale sur l'équilibre physiologique du patient.

II est donc important de ne délivrer de tels chocs que de façon parfaitement justifiée.

Diverses techniques ont été proposées pour réduire l'incidence du bruit, notamment l'adaptation automatique de la sensibilité des amplificateurs de détection, ou le contrôle automatique du gain de ces amplificateurs.

Mais, d'une part, pour détecter la fibrillation ventriculaire (FV), dont le niveau du signal est faible, il est nécessaire de disposer d'une sensibilité maximale, sous peine de ne pas détecter des événements qui auraient dû l'être ("sous-détections").

Toutefois, l'amplitude des signaux de fibrillation ventriculaire (les complexes QRS représentatifs des dépolarisations du ventricule) peut se situer à un niveau variable, intermédiaire entre le niveau du bruit et celui des signaux des complexes sinusaux.

Si l'on veut pouvoir détecter la fibrillation ventriculaire, la détection d'un bruit éventuel est donc inévitable. De ce fait :
- si l'implant est programmé à une valeur de sensibilité trop élevée, c'est-à-dire avec un seuil de détection trop bas, les épisodes d'arythmie ventriculaire seront convenablement détectés, mais la sensibilité accrue aux bruits augmente fortement les risques d'application d'une thérapie inappropriée et indésirable (situation de faux positif, dite *oversensing*) ;
- inversement, si l'implant est programmé à une valeur de sensibilité trop faible, c'est-à-dire avec un seuil de détection trop élevé, les épisodes réels d'arythmie ventriculaire risquent de ne pas être détectés (situation de faux négatif, dite de "sous-détection"), avec pour le patient des conséquences qui peuvent être très graves.

La détection de bruits extrinsèques étant généralement inévitable, le problème de l'invention consiste à ajuster les paramètres de détection de l'arythmie ventriculaire de manière à discriminer ces bruits parmi les dépolarisations cardiaques, ceci afin d'éviter le déclenchement de traitements inappropriés ou, inversement, l'inhibition de traitements qui auraient été justifiés.

Diverses propositions ont été formulées dans l'état de la technique pour tenter de trouver une solution à ce problème.

Le US 2011/0172727 propose, en cas d'arythmie probable, d'exécuter un algorithme de suspicion de détection inappropriée, basé sur le nombre des intervalles RR courts détectés, le couplage entre les cavités (stabilité de l'intervalle PR), la morphologie des signaux de dépolarisation ventriculaire et l'instabilité du couplage RR. En cas de détection effectivement suspecte, le dispositif augmente la persistance, c'est-à-dire le temps de latence pour l'application d'une thérapie, ou modifie la thérapie appliquée (réduction du nombre de chocs, ou modification de la séquence de stimulation ATP). L'inconvénient de cette technique tient au fait que, en cas de détection de bruit, les conséquences d'une thérapie inappropriée sont seulement réduites, mais cette thérapie n'est pas empêchée. En outre, la technique de discrimination divulguée ne fonctionne pas en cas de bruit soutenu de forte amplitude.

Le US 7 953 488 propose, en cas de bruit détecté ou de sous-détection ventriculaire, d'effectuer une étape de confirmation en opérant une commutation de la voie de détection pour vérifier que le bruit est absent sur cette autre voie. Cette technique ne fonctionne évidemment que dans une situation où le bruit n'est présent que sur une seule des deux voies ; dans le cas contraire, le risque d'application d'une thérapie inappropriée reste entier.

Le US 7 937 135 prévoit d'ajuster la sensibilité de détection en fonction de l'amplitude du signal, analysée sur deux voies ECG. En cas de suspicion de bruit, une fenêtre est ouverte pendant laquelle sont analysées la morphologie et l'amplitude du signal. Si du bruit est détecté, la fréquence mesurée est corrigée (les couplages courts sont invalidés) pour éviter un diagnostic inapproprié. Cette technique ne convient pas en cas de bruit d'amplitude élevée ; en outre, elle augmente le risque de non-détection d'une fibrillation ventriculaire en cas d'erreur sur la discrimination entre bruit et FV.

Le US 7 756 570 prévoit d'augmenter la sensibilité de détection en l'absence de bruit, et de la réduire dès que du bruit est détecté. Au bout d'une période prédéterminée (un ou plusieurs cycles stimulés), la sensibilité revient à sa valeur minimale, pour vérifier si du bruit est toujours présent ou si une arythmie n'est pas survenue entretemps (le seuil de détection étant fonction de l'amplitude de l'onde R). Le seuil d'amplitude pour la détection du bruit est ainsi un seuil adaptatif, variable, mis à jour en fonction du niveau du bruit détecté. L'inconvénient de cette technique est le retard qu'elle introduit dans la détection d'une arythmie (du fait de l'application d'une sensibilité élevée après une période de temps fixe), avec un risque de détecter des cycles bruités au moment où la sensibilité élevée est rétablie (situation de pause ventriculaire).

Le US 2002/0165587 A1 opère de façon comparable, en déterminant à chaque cycle un niveau de bruit et un niveau de signal et en ajustant le seuil de détection en fonction de ces niveaux respectifs, mais avec les mêmes inconvénients que précédemment. Le US 2004/0015192 A1 prévoit également d'utiliser un seuil dynamique variable pour améliorer les conditions de délivrance d'un choc de défibrillation.

D'autres techniques encore ont été proposées pour réduire l'incidence des bruits électromagnétiques parasites: ainsi, le US 2011/0196247 A1 opère un comptage des fluctuations du signal dans une fenêtre temporelle définie entre deux événements cardiaques, et estime la présence éventuelle de bruit en fonction du résultat de ce comptage. Le US 2009/0326600 A1, quant à lui, prévoit en cas d'arythmie suspectée une mesure d'impédance des électrodes afin de discriminer entre arythmie et bruit.

Le but de l'invention est de proposer une technique de détection des arythmies ventriculaires permettant de pallier ces limitations et difficultés, en évitant la détection du bruit ventriculaire tout en assurant la bonne détection des arythmies ventriculaires.

L'idée de base de l'invention consiste, essentiellement, à appliquer une sensibilité ventriculaire faible (à augmenter le seuil de détection) en cas de suspicion de bruit. De ce fait, l'implant déclenchera une stimulation en cas de sous-détection liée à cette sensibilité abaissée. Or, si cette stimulation survient pendant une arythmie, elle ne sera pas efficace - ce que l'on peut vérifier par un test de capture, c'est-à-dire de détection d'une dépolarisation induite par la stimulation qui vient d'être appliquée. En fonction du résultat de ce test : (i) soit la sensibilité sera maintenue basse (stimulation efficace, révélant une absence d'arythmie), (ii) soit la sensibilité sera réajustée à un niveau élevé pour éviter le risque de sous-détection -car dans ce cas le bruit suspecté est en fait un épisode d'arythmie ventriculaire (stimulation inefficace du fait de l'arythmie présente).

Plus précisément, l'invention a pour objet un dispositif médical actif de type défibrillateur/cardioverteur implantable comprenant, de manière en elle-même connue, par exemple d'après le US 2011/0196247 A1 précité : des moyens de recueil de l'activité électrique du coeur ; des moyens d'analyse, pour détecter la survenue d'événements ventriculaires spontanés à partir de l'activité électrique du coeur, ces moyens d'analyse opérant avec une sensibilité fonction d'un seuil ajustable de détection ; des moyens d'ajustement du seuil de détection des moyens d'analyse ; des moyens de stimulation, aptes à délivrer une impulsion de stimulation en cas d'absence d'événement ventriculaire détecté à l'issue d'un intervalle d'échappement prédéterminé ; des moyens de détection de la présence d'arythmies dans les événements ventriculaires spontanés détectés par les moyens d'analyse ; et des moyens d'application éventuelle d'une thérapie antitachycardique en cas d'apparition desdites arythmies. Ce dispositif comporte en outre des moyens de détection de bruit, aptes à délivrer un signal de suspicion de bruit fonction de l'activité électrique recueillie, et des moyens de test de capture, aptes à détecter la survenue d'une dépolarisation induite consécutive à l'application d'une impulsion de stimulation.

De façon caractéristique de l'invention, les moyens d'ajustement du seuil de détection sont des moyens aptes, sur détection de bruit, à relever le niveau du seuil de détection jusqu'à un niveau de masquage du bruit détecté et, sur délivrance d'une impulsion de stimulation, à abaisser le niveau du seuil de détection en l'absence de dépolarisation induite détectée par les moyens de test de capture, et maintenir le niveau du seuil de détection en présence d'une dépolarisation induite détectée par les moyens de test de capture.

Le relèvement du niveau de seuil de détection sur détection de bruit peut être soit graduel, par pas successifs sur la durée d'une période réfractaire redéclenchable, soit directement à un niveau fonction de l'amplitude des cycles pour lesquels du bruit a été détecté.

L'abaissement du niveau de seuil de détection en l'absence de dépolarisation induite peut être soit graduel, par pas successifs jusqu'à une valeur limite préprogrammée, soit directement à une valeur limite préprogrammée.

On va décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est un ensemble de chronogrammes illustrant l'incidence d'épisodes de bruit ventriculaire de forte amplitude sur la survenue de fausses détections, avec des implants selon l'état de la technique.

La Figure 2 est un ensemble de chronogrammes illustrant un épisode typique de fibrillation ventriculaire avec des signaux de faible amplitude.

La Figure 3 est un organigramme montrant la séquence des différentes étapes mises en oeuvre par l'invention.

La Figure 4 est un ensemble de chronogrammes illustrant le comportement d'un implant selon l'invention en cas d'apparition d'un épisode de bruit ventriculaire.

La Figure 5 est un ensemble de chronogrammes illustrant le comportement de l'implant selon l'invention en cas de survenue d'un épisode d'arythmie ventriculaire de faible amplitude.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Ovatio* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous.

Le procédé de l'invention est avantageusement mis en oeuvre par des moyens principalement logiciels, au moyen d'algorithmes appropriés exécutés par un microcontrôleur ou un processeur numérique de signal. L'adaptation de l'implant à la mise en oeuvre des fonctions de l'invention est à la portée de l'homme du métier, et de ce fait elle ne sera pas décrite en détail.

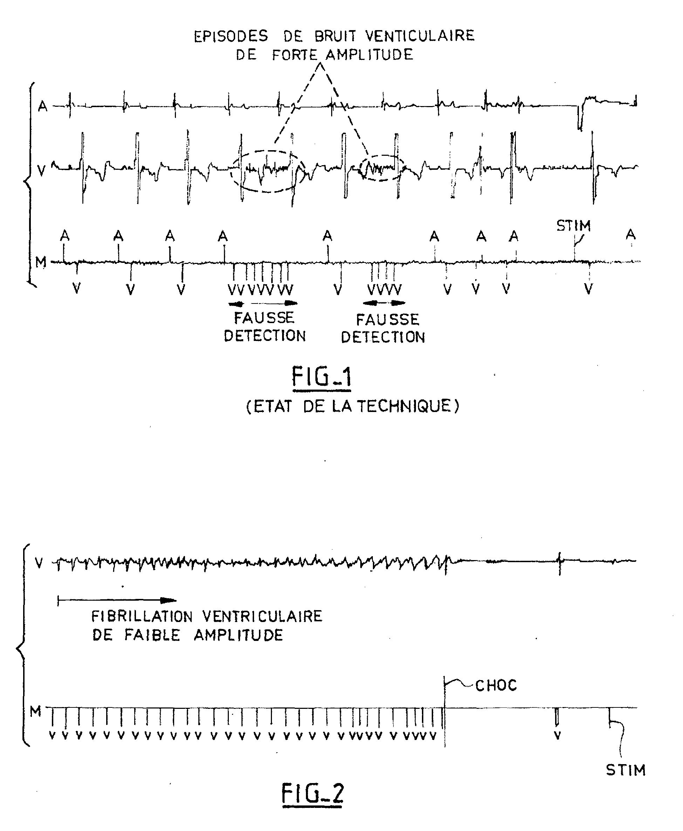

Sur la Figure 1, les deux premiers chronogrammes illustrent des signaux d'électrogramme (EGM) respectivement auriculaire (A) et ventriculaire (V) recueillis par un implant.

La sensibilité de détection ventriculaire, programmée par le médecin au moment de l'implantation ou ultérieurement, est généralement fixée à un niveau de l'ordre de 0,4 mV. Le chronogramme du bas illustre les marqueurs (M) correspondants délivrés par l'algorithme d'analyse de ces signaux EGM, avec des marqueurs de détection auriculaire (A), ventriculaire (V) ainsi que des marqueurs d'application d'une stimulation déclenchée par l'implant (STIM).

On notera la présence sur le signal ventriculaire de deux épisodes de bruit ventriculaire de forte amplitude, c'est-à-dire d'amplitude typiquement supérieure à 1 mV, pouvant même aller jusqu'à 5 mV. Un tel bruit est interprété - à tort - par l'implant comme une fibrillation ventriculaire (succession de marqueurs V très rapprochés), ce qui pourrait inhiber la stimulation, créant une pause ventriculaire pour les patients dépendants, et également, s'il perdure, déclencher pour le patient une thérapie inappropriée.

La Figure 2 illustre une situation typique de véritable fibrillation ventriculaire, avec sur le signal V une succession de dépolarisations très rapides. Après analyse par l'algorithme, celui-ci décide de déclencher une thérapie par application d'un choc de défibrillation, ce qui a pour conséquence le retour à un rythme sinusal normal, si besoin avec stimulation du ventricule.

Une difficulté particulière se présente lorsque cette fibrillation ventriculaire présente une faible amplitude, avec un niveau du signal ventriculaire détecté de l'ordre de 2 à 5 mV.

Pour être sûr de bien détecter de telles arythmies, il est important de programmer l'implant avec une sensibilité élevée (seuil de détection faible), donc avec un risque plus élevé de détecter du bruit ventriculaire, ce qui pourrait conduire à déclencher des thérapies inappropriées et délétères pour le patient.

L'invention propose une technique permettant d'abaisser sans risque cette sensibilité (pour une meilleure immunité au bruit) tout en conservant une bonne détection des arythmies.

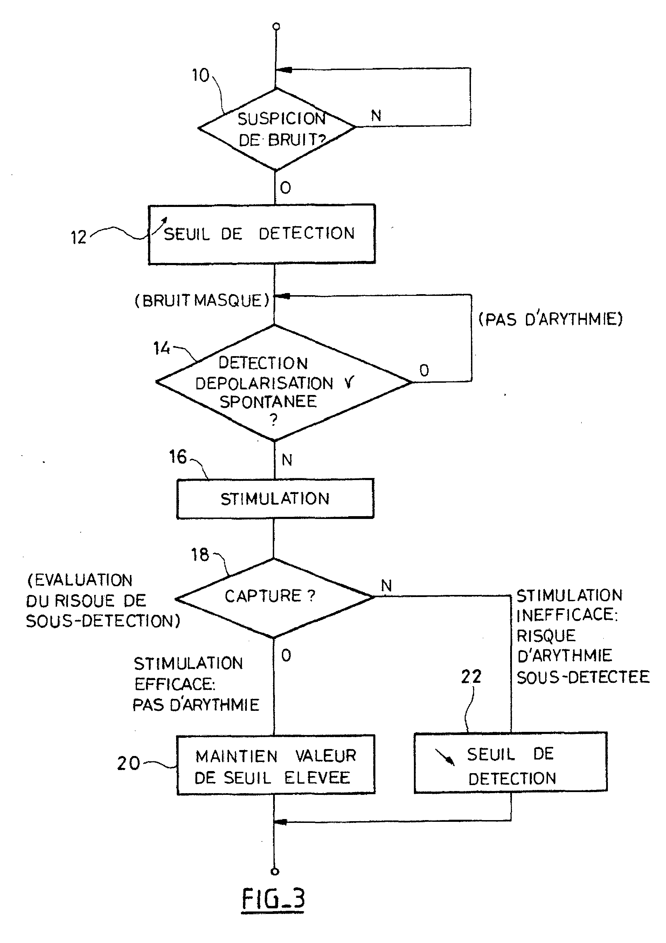

Les étapes mises en oeuvre par l'invention sont illustrées sur l'organigramme de la Figure 3.

La première étape (bloc 10) est un test de suspicion de bruit. Il est possible de mettre en oeuvre à cet effet diverses techniques connues, par exemple celles décrites dans les documents suivants :
- EP 0 838 235 A1 (ELA Medical) qui décrit un algorithme de détection et de classification des tachyarythmies mis en oeuvre notamment sous la dénomination *PARAD*/*PARAD+* dans les appareils *Ovatio* et *Paradym* de Sorin CRM : lorsqu'une arythmie est détectée, le bruit ventriculaire est suspecté du fait de la présence de cycles courts dus à des artefacts et non à une vraie arythmie ;
- EP 1 857 142 A1, qui opère une double détection : de la dépolarisation par analyse du signal électrique EGM, et de la contraction du myocarde par mesure de l'accélération endocardiaque via un accéléromètre directement en contact avec le muscle cardiaque. Il y aura suspicion de bruit lorsque le dispositif détectera un signal électrique (sensible au bruit) non suivi d'une contraction (phénomène mécanique qui n'est pas affecté par le bruit) ;
- EP 2 368 493 A1 (ELA Medical), qui prévoit d'analyser simultanément deux voies EGM différentes, à savoir celles d'un signal unipolaire et d'un signal bipolaire. Cette analyse est une analyse bidimensionnelle à partir de la "boucle cardiaque" ou vectogramme VGM, qui est la représentation dans un espace à deux dimensions de l'un des deux signaux par rapport à l'autre. Les caractéristiques morphologiques du vectogramme sont analysées de manière à discriminer entre dépolarisation effective et bruit recueilli par les circuits de détection.

En cas de bruit suspecté, le seuil de détection est relevé (bloc 12), ce qui diminue la sensibilité. Cette augmentation peut être progressive et régulière, par exemple une augmentation de 0,2 mV toutes les 16 ms tant que la période réfractaire redéclenchable est en cours, afin de passer au-dessus de l'amplitude du bruit. Une autre possibilité consiste à calculer une valeur cible de seuil de détection en prenant par exemple sur six cycles la moyenne de l'amplitude des cycles détectés comme bruités ; le nouveau seuil appliqué correspondra à cette amplitude moyenne, limitée à une valeur maximale.

L'élévation du seuil de détection permet ainsi de couvrir le bruit dont la présence avait été détectée à l'étape précédente.

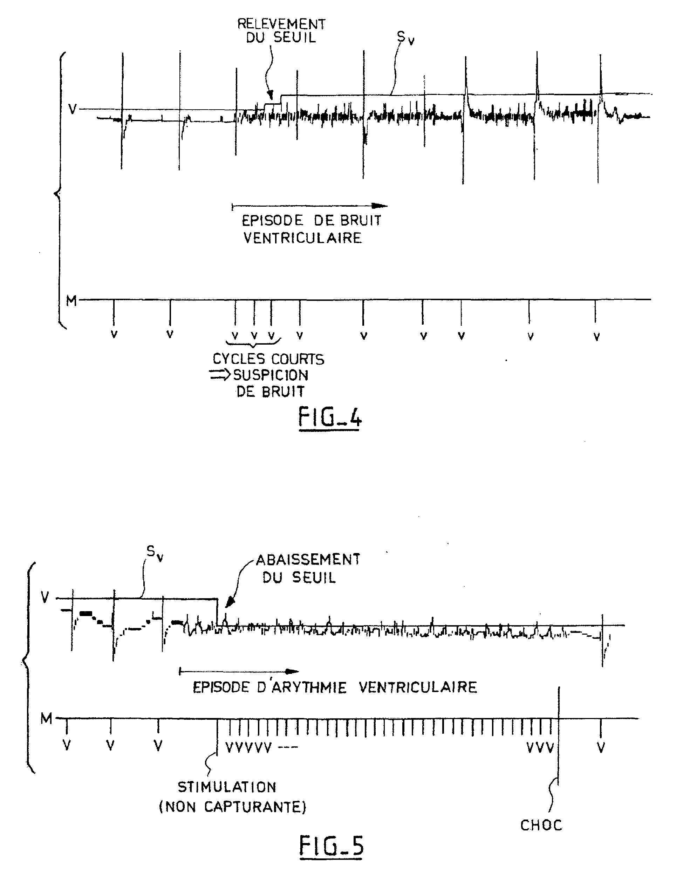

La Figure 4 illustre cette situation : la survenue d'un épisode de bruit ventriculaire entraîne l'apparition de cycles détectés comme courts (marqueurs V), en fait constitués d'artefacts. Le seuil de détection ventriculaire S_{V} est alors relevé, ce qui a pour conséquence de faire disparaître ces artefacts : seuls subsistent alors les marqueurs correspondant aux véritables dépolarisations ventriculaires.

Il importe toutefois que le bruit ainsi masqué ne conduise pas à une sous-détection.

De fait, la sensibilité de l'implant ayant été maintenant réduite, deux possibilités se présentent :
- l'implant détecte une dépolarisation ventriculaire spontanée (bloc 14) : ceci signifie qu'il n'y a pas de FV, car on est certain qu'il s'agit là d'une véritable dépolarisation, au-dessus du niveau du bruit ;
- dans le cas contraire, l'absence de dépolarisation ventriculaire spontanée détectée à la fin de l'intervalle d'échappement conduit l'implant à appliquer au ventricule une impulsion de stimulation (bloc 16). Il peut s'agir d'une stimulation normale, requise compte tenu de l'état du patient ; mais il peut également s'agir d'une situation de sous-détection d'une arythmie ventriculaire, en cas de FV d'amplitude faible, inférieure au seuil qui avait été relevé à l'étape 12.

Pour vérifier que dans ce dernier cas on ne sous-détecte pas une arythmie ventriculaire, on procède alors à un test de capture (bloc 18) sur le cycle stimulé. Ce test de capture peut être effectué par l'une de diverses méthodes connues, décrites par exemple dans les documents suivants :
- WO 93/02741 A1 et EP 1 287 849 A1 (ELA Medical) : par analyse des signaux électriques recueillis (analyse de la réponse évoquée) ;
- EP 2 324 885 A1 (Sorin CRM) : par analyse du vectogramme cardiaque VGM, à partir de paramètres descripteurs de la caractéristique bidimensionnelle non temporelle obtenue ;
- EP 2 412 401 A1 (Sorin CRM), publié le 01.02.2012 : par détection d'une activité mécanique du coeur consécutive à la stimulation.

Si la capture est avérée, cela signifie que la stimulation est efficace et que le patient n'est pas en arythmie. L'implant peut donc alors maintenir la sensibilité réduite (étape 20), avec le seuil au niveau où il avait été relevé à l'étape 12.

Dans le cas contraire, l'absence de capture indique que la stimulation n'est pas efficace, vraisemblablement du fait d'une arythmie en cours. Pour éviter toute sous-détection de cette arythmie, l'implant va augmenter la sensibilité de détection (étape 22) par abaissement du seuil, soit graduel (par exemple par pas de 0,2 mV toutes les 16 ms) jusqu'à la sensibilité programmée par le médecin, soit directement à cette valeur de sensibilité programmée.

La Figure 5 illustre cette situation : au début d'un épisode d'arythmie ventriculaire, l'implant ne détecte plus le rythme régulier des dépolarisations ventriculaires (marqueurs V) et déclenche donc une stimulation. Cette stimulation n'étant pas suivie d'une capture (capture détectée par l'un des moyens exposés plus haut), le seuil de détection ventriculaire S_{V} est alors abaissé, et ceci à un niveau permettant de détecter l'activité électrique caractéristique de l'arythmie ventriculaire (succession de marqueurs V à intervalles très rapprochés), conduisant à l'application - ici justifiée - d'un choc de défibrillation.

## Revendications

1. Un dispositif médical actif de type défibrillateur/cardioverteur implantable, comprenant :
- des moyens de recueil de l'activité électrique du coeur ;
- des moyens d'analyse, pour détecter la survenue d'événements ventriculaires spontanés à partir de l'activité électrique du coeur, ces moyens d'analyse opérant avec une sensibilité fonction d'un seuil ajustable de détection ;
- des moyens d'ajustement du seuil de détection des moyens d'analyse ;
- des moyens de stimulation, aptes à délivrer une impulsion de stimulation en cas d'absence d'événement ventriculaire détecté à l'issue d'un intervalle d'échappement prédéterminé ;
- des moyens de détection de la présence d'arythmies dans les événements ventriculaires spontanés détectés par les moyens d'analyse ; et
- des moyens d'application éventuelle d'une thérapie antitachycardique en cas d'apparition desdites arythmies ;
- des moyens de détection de bruit, aptes à délivrer (10) un signal de suspicion de bruit fonction de l'activité électrique recueillie ; et
- des moyens de test de capture, aptes à détecter (18) la survenue d'une dépolarisation induite consécutive à l'application d'une impulsion de stimulation,
**caractérisé en ce que** les moyens d'ajustement du seuil de détection sont des moyens aptes :
- sur détection de bruit, à relever (12) le niveau du seuil de détection jusqu'à un niveau de masquage du bruit détecté ; et
- sur délivrance (16) d'une impulsion de stimulation, à :
• abaisser (22) le niveau du seuil de détection en l'absence de dépolarisation induite détectée par les moyens de test de capture, et
• maintenir (20) le niveau du seuil de détection en présence d'une dépolarisation induite détectée par les moyens de test de capture.

2. Le dispositif de la revendication 1, dans lequel le relèvement du niveau de seuil de détection sur détection de bruit est un relèvement graduel, par pas successifs sur la durée d'une période réfractaire redéclenchable.

3. Le dispositif de la revendication 1, dans lequel le relèvement du niveau de seuil de détection sur détection de bruit est un relèvement directement à un niveau fonction de l'amplitude des cycles pour lesquels du bruit a été détecté.

4. Le dispositif de la revendication 1, dans lequel l'abaissement du niveau de seuil de détection en l'absence de dépolarisation induite est un abaissement graduel, par pas successifs jusqu'à une valeur limite préprogrammée.

5. Le dispositif de la revendication 1, dans lequel l'abaissement du niveau de seuil de détection en l'absence de dépolarisation induite est un abaissement directement à une valeur limite préprogrammée.

## Patentansprüche

1. Aktive medizinische Vorrichtung des Typs implantierbarer Defibrillator/Kardioverter, die Folgendes umfasst:
- Mittel zum Erfassen der elektrischen Aktivität des Herzens;
- Analysemittel, um den Eintritt von spontanen ventrikulären Ereignissen anhand der elektrischen Aktivität des Herzens zu detektieren, wobei diese Analysemittel mit einer Empfindlichkeit arbeiten, die eine Funktion eines einstellbaren Detektionsschwellenwerts ist;
- Mittel zum Einstellen des Detektionsschwellenwerts der Analysemittel;
- Stimulationsmittel, die dafür ausgelegt sind, einen Stimulationsimpuls bei Abwesenheit eines detektierten ventrikulären Ereignisses am Ende eines vorgegebenen Ausreissintervalls zu verabreichen;
- Mittel zum Detektieren des Vorhandenseins von Arrhythmien in den durch die Analysemittel detektierten spontanen ventrikulären Ereignissen; und
- Mittel zum eventuellen Anwenden einer Antitachykardie-Therapie im Fall des Auftretens von Arrhythmien;
- Mittel zum Detektieren von Rauschen, die dafür ausgelegt sind, ein Rauschverdachtssignal, das eine Funktion der erfassten elektrischen Aktivität ist, zu verabreichen (10); und
- Aufnahmetestmittel, die dafür ausgelegt sind, das Auftreten einer induzierten Depolarisation als Folge des Anwendens eines Stimulationsimpulses zu detektieren (18),
**dadurch gekennzeichnet, dass** die Mittel zum Einstellen des Detektionsschwellenwerts Mittel sind, die dafür ausgelegt sind:
- bei Detektion von Rauschen den Detektionsschwellenpegel bis auf einen Pegel zum Maskieren des detektierten Rauschens anzuheben (12); und
- bei Verabreichung (16) eines Stimulationsimpulses:
• den Detektionsschwellenpegel bei Abwesenheit einer durch die Aufnahmetestmittel detektierten induzierten Depolarisation zu senken (22), und
• den Detektionsschwellenpegel bei Anwesenheit einer durch die Aufnahmetestmittel detektierten induzierten Depolarisation aufrecht zu erhalten (20).

2. Vorrichtung nach Anspruch 1, wobei das Erhöhen des Detektionsschwellenpegels bei Detektion von Rauschen ein allmähliches Anheben in aufeinander folgenden Schritten während der Dauer einer erneut auslösbaren Refraktärperiode ist.

3. Vorrichtung nach Anspruch 1, wobei das Anheben des Detektionsschwellenpegels bei Detektion von Rauschen ein direktes Anheben auf einen Pegel, der eine Funktion der Amplitude der Zyklen ist, für die Rauschen detektiert worden ist, ist.

4. Vorrichtung nach Anspruch 1, wobei das Absenken des Detektionsschwellenpegels bei Abwesenheit einer induzierten Depolarisation ein allmähliches Absenken in aufeinander folgenden Schritten bis auf einen vorprogrammierten Grenzwert ist.

5. Vorrichtung nach Anspruch 1, wobei das Absenken des Detektionsschwellenpegels bei Abwesenheit einer induzierten Depolarisation ein direktes Absenken auf einen vorprogrammierten Grenzwert ist.

## Claims

1. An active medical device of the implantable defibrillator/cardioverter type, comprising:
- means for collecting the electrical activity of the heart;
- analysis means for detecting the occurrence of spontaneous ventricular events based on the electrical activity of the heart, these analysis means operating with a sensitivity that is function of an adjustable detection threshold;
- means for adjusting the detection threshold of the analysis means;
- stimulation means adapted to deliver a stimulation pulse in case of absence of ventricular event detected at the end of a predetermined escape interval;
- means for detecting the presence of arrhythmias in the spontaneous ventricular events detected by the analysis means; and
- means for possibly applying an anti-tachycardia therapy in case of appearance of said arrhythmias;
- noise detection means adapted to deliver (10) a noise suspicion signal that is function of the electrical activity collected; and
- capture test means adapted to detect (18) the occurrence of an induced depolarization consecutive to the application of a stimulation pulse,
**characterized in that** the detection threshold adjustment means are means adapted to:
- upon noise detection, increase (12) the detection threshold level to a level masking the detected noise; and
- upon delivery (16) of a stimulation pulse:
. lowering (22) the detection threshold level in the absence of induced depolarization detected by the capture test means, and
. maintaining (20) the detection threshold level in the presence of an induced depolarization detected by the capture test means.

2. The device of claim 1, wherein the increasing of the detection threshold level upon detection of noise is a gradual increasing, by successive steps over the duration of a retriggerable refractory period.

3. The device of claim 1, wherein the increasing of the detection threshold level upon detection of noise is an increasing directly to a level that is function of the amplitude of the cycles for which the noise has been detected.

4. The device of claim 1, wherein the lowering of the detection threshold level in the absence of induced depolarization is a gradual lowering, by successive steps, down to a pre-programmed limit value.

5. The device of claim 1, wherein the lowering of the detection threshold level in the absence of induced depolarization is a lowering directly down to a pre-programmed limit value.
